# EUROPÄISCHE PATENTSCHRIFT

(11) **EP 3 649 113 B1**
(45) Veröffentlichungstag und Bekanntmachung des Hinweises auf die Patenterteilung: **18.03.2026**
(21) Anmeldenummer: 18733647.4
(22) Anmeldetag: 02.07.2018
(51) Int. Cl.: C07D 251/34, C08G 18/79, C08G 18/02, C09D 175/04, C09J 175/04

(54) **KONTINUIERLICHE VERDÜNNUNG VON POLYISOCYANATEN**
CONTINUOUS DILUTION OF POLYISOCYANATES
DILUTION CONTINUE DE POLYISOCYANATES

(30) Priorität: 05.07.2017 EP 17179716; 27.04.2018 EP 18169695
(43) Veröffentlichungstag der Anmeldung: 13.05.2020
(73) Patentinhaber: Covestro Deutschland AG, 51373 Leverkusen (DE)
(72) Erfinder: GROTH, Stefan, 51375 Leverkusen (DE); WU, Ruiwen, 51375 Leverkusen (DE); MIDOLO, Antonio, 50679 Köln (DE)
(74) Vertreter: Levpat
(86) Internationale Anmeldenummer: PCT/EP2018/067787
(87) Internationale Veröffentlichungsnummer: WO 2019/007895

(56) Entgegenhaltungen:
- EP-A1- 3 476 826
- WO-A1-2014/139873
- WO-A1-2014/139879
- WO-A1-2017/021150
- US-A- 4 419 513
- US-A- 5 086 175
- ALMAT� MARIA ET AL: "New innovative high- performance hardeners from Covestro: efficiency, sustainability and more", 13 October 2016 (2016-10-13), pages 1 - 26, XP055908163, Retrieved from the Internet <URL:https://www.ramspec.eu/wp-content/uploads/2016/10/2_Covestro.pdf> [retrieved on 20220401]
- S.A.P.I.C.I. SPA: "Polurene HR.B.S", 1 February 2010 (2010-02-01), XP002774804, Retrieved from the Internet <URL:http://www.twanfong.com/product/01_hardener/01-11/HR.B.S.pdf> [retrieved on 20171017]
- S.A.P.C.I. SPA: "Polurene OK-HP", 14 January 2013 (2013-01-14), XP002774805, Retrieved from the Internet <URL:https://www.palmerholland.com/Assets/User/Documents/Product/43997/3175/MITM08281.PDF> [retrieved on 20171017]
- S.A.P.I.C.I. SPA: "Polurene 3031", 1 January 2010 (2010-01-01), XP002774806, Retrieved from the Internet <URL:http://www.twanfong.com/product/01_hardener/01-11/3031.pdf> [retrieved on 20171017]
- VENCOREX CHEMICALS: "Easaqua X L 600", March 2016 (2016-03-01), XP002774807, Retrieved from the Internet <URL:http://www.vencorex.com/wp-content/uploads/2016/05/PDS-Easaqua%E2%84%A2-X-L-600-March-2016-ENG.pdf> [retrieved on 20171017]

## Beschreibung

Die vorliegende Erfindung betrifft ein Verfahren zur Herstellung einer Polyisocyanatzusammensetzung aus Toluylendiisocyanat, und die daraus erhältlichen Polyisocyanatzusammensetzungen. Außerdem betrifft die Erfindung die Verwendung einer kontinuierlichen Verdünnung

Urethangruppen aufweisende Polyisocyanate aus Polyhydroxyverbindungen und Toluylendiisocyanat sind seit langem bekannt und werden z.B. in der DE 870 400, DE 953 012 und DE 1 090 196 beschrieben.

Isocyanurate des Toluylendiisocyanats werden durch Cyclotrimerisierung unter Verwendung verschiedener Katalysatoren hergestellt. Solche Umsetzungsprodukte sind ebenfalls seit langem bekannt und werden zum Beispiel in der DE 951168 B, DE 1013869 A, US 6,936,678 B2, DE 19523657 A1, US 4255569 A, EP 2 174 967 B1 und der CN 105001701 beschrieben.

US 5 086 175 A offenbart Polyisocyanatzusammensetzungen mit einer Konzentration von 75%, die ein Polyisocyanat und ein Lösungsmittel enthalten sowie frei sind von Trübungen.

US 4 419 513 A offenbart Polyisocyanate aus Mischungen von Hexamethylendiisocyanat und 1-lsocyanato-3,3,5-trimethyl-5-isocyanatomethylcyclohexan, die, um überschüssiges Monomer zu entfernen, nach der Herstellung einer Destillation unterzogen und danach mit einem Lösungsmittel verdünnt werden.

Seit langem besteht der Wunsch, die bekannten Isocyanurate des Toluylendiisocyanats einerseits niedrig-viskos, andererseits hoch-funktionell darzustellen. Toluylendiisocyanat wird im Folgenden auch als TDI bezeichnet.

Eine niedrige Viskosität ist zum Beispiel erwünscht, um das Applikationsverhalten von Lacken und Klebstoffen zu verbessern. Daneben kann bei Verwendung von niedrig-viskosen Polyisocyanaten als Vernetzer von Lacken und Klebstoffen der Lösemittelgehalt der Formulierung gesenkt werden. Das bedeutet, dass die Emissionen flüchtiger organischer Verbindungen solcher Formulierungen reduziert werden können, ohne die Anwendbarkeit negativ zu beeinflussen.

Außerdem ist es beim Einsatz solcher Polyisocyanate als Vernetzer in Lacken und Klebstoffen erwünscht, dass die Polyisocyanate einen hohen Gehalt an Isocyanatgruppen aufweisen. Dadurch wird die Nachhaltigkeit im Sinne eines niedrigen Gehalts an organischen Lösungsmitteln und schneller Vernetzung, also hoher Prozesseffizienz, weiter gesteigert.

Daneben ist es erwünscht, dass die Isocyanurate des TDI einen niedrigen Gehalt an freiem Diisocyanat enthalten. Wegen der toxikologischen Bedenklichkeit des monomeren TDI ist dies eine wichtige Bedingung für die universelle Einsetzbarkeit in industriell applizierten Lacken und Klebstoffen.

Bei der Umsetzung von TDI zum Polyisocyanurat werden, wie aus DE 951168B, DE 1013869A bekannt, sehr hoch-viskose Harze erhalten, was die Verarbeitung erschwert oder es erforderlich macht, größere Mengen organischer Lösungsmittel zu verwenden. Daneben weisen Polyisocyanate des TDI eine hohe Tendenz zur Kristallisation auf und sind nur schwer in organischen Lösungsmitteln löslich.

Eine weitere wichtige Rahmenbedingung stellt die Löslichkeit der oligomeren Isocyanurate in gängigen organischen Lösungsmitteln dar. Eine unvollständige Löslichkeit führt zu trüben Lösungen, was die Verwendbarkeit in Lack- oder Klebstoff-Formulierungen stark einschränkt.

Aufgabe der vorliegenden Erfindung war es daher, ein Verfahren zur Herstellung einer Polyisocyanatzusammensetzung bereitzustellen, mit dem sich dem sich Polyisocyanatzusammensetzungen erhalten lassen, die eine niedrige Viskosität bei gleichzeitig möglichst hohem Gehalt an Isocyanatgruppen aufweisen. Zusätzlich sollten die Polyisocyanatzusammensetzungen eine möglichst geringe Farbzahl aufweisen.

Diese Aufgabe wurde gelöst durch Verfahren zur Herstellung einer Polyisocyanatzusammensetzung wie es im Anspruch 1 definiert ist.

Mit dem erfindungsgemäßen Verfahren lassen sich Polyisocyanatzusammensetzungen herstellen, die gegenüber dem Stand der Technik trübungsstabiler sind und daher auch für Verwendungen geeignet sind, in denen klare Lösungen benötigt werden. Weiter verbessert wird der Einsatzbereich dadurch, dass die Polyisocyanatzusammensetzungen eine Farbzahl von < 100 Hazen, bevorzugt < 95 Hazen aufweisen. Die Farbzahl in Hazen wird nach DIN EN 1557:1997-03 bestimmt.

Somit ist auch ein Verfahren zur Herstellung einer Polyisocyanatzusammensetzung, die eine nach DIN EN 1557:1997-03 bestimmte Farbzahl von < 100 Hazen, bevorzugt < 95 Hazen aufweist, umfassend die Zugabe mindestens eines gegenüber Isocyanatgruppen inerten Lösungsmittels zu mindestens einem Polyisocyanat, dadurch gekennzeichnet, dass die Zugabe des Lösungsmittels in einer oder mehrerer Stufen erfolgt und mindestens eine dieser Stufen als kontinuierliche Verdünnung durchgeführt wird, eine vorteilhafte Ausführungsform der vorliegenden Erfindung.

Offenbarungsgemäß, aber nicht unter den Schutzumfang der Ansprüche fallend, ist das Polyisocyanat ein Polyisocyanat auf Basis von mindestens einem aliphatischen, cycloaliphatischen araliphatischen oder aromatischen Diisocyanat außer Toluylendiisocyanat (TDI), bevorzugt aus einem araliphatischen oder aromatischen Diisocyanat außer Toluylendiisocyanat (TDI). Der Begriff *aus einem Diisocyanat* ist gleichbedeutend mit *auf Basis eines solchen Diisocyanats.*

Offenbarungsgemäß geeignete, aber nicht unter den Schutzumfang des Anspruchs fallende, aliphatische, cycloaliphatische, araliphatische oder aromatische Diisocyanate sind beispielsweise ausgewählt aus der Gruppe bestehend aus 1,4-Diisocyanatobutan, 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 2,4- und 2,6-Diisocyanato-1-methylcyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, 1-Isocyanato-1-methyl-4(3)isocyanato-methylcyclohexan, Bis-(isocyanatomethyl)-norbornan, 1,3- und 1,4-Bis(isocyanatomethyl)benzol (XDI), 1,3- und 1,4-Bis-(2-isocyanato-prop-2-yl)-benzol (TMXDI), 2,4'- und 4,4'-Diisocyanatodiphenylmethan (MDI), 1,5-Diisocyanatonaphthalin, 1,3- und 1,4-Phenylendiisocyanat, oder beliebige Gemische solcher Diisocyanate.

Offenbarungsgemäß bevorzugte, aber nicht unter den Schutzumfang des Anspruchs fallende, Diisocyanate sind hierbei 1,5-Diisocyanatopentan (PDI), 1,6-Diisocyanatohexan (HDI), 1,3- und 1,4-Diisocyanatocyclohexan, 2,4- und 2,6-Diisocyanato-1-methylcyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, 1,3- und 1,4-Bis(isocyanatomethyl)benzol (XDI), 2,4'- und 4,4'-Diisocyanatodiphenylmethan (MDI) oder 1,5-Diisocyanatonaphthalin.

Insbesondere lag der vorliegenden Erfindung die spezielle Aufgabe zugrunde ein Verfahren zur Herstellung einer Polyisocyanatzusammensetzung aus Toluylendiisocyanat bereitzustellen, mit dem sich Polyisocyanatzusammensetzungen aus Toluylendiisocyanat erhalten lassen, die eine niedrige Viskosität bei gleichzeitig möglichst hohem Gehalt an Isocyanatgruppen aufweisen und zudem als trübungsstabile Lösungen vorliegen. Zusätzlich sollten die Polyisocyanat-zusammensetzungen eine möglichst geringe Farbzahl aufweisen.

Diese spezielle Aufgabe wurde gelöst durch ein Verfahren wie es in Anspruch 1 definiert ist.

Somit ist in der Erfindung das Polyisocyanat auf Basis von Toluyendiisocyanat. Der Begriff "aus Toluylendiisocyanat" ist gleichbedeutend mit "auf Basis von Toluylendiisocyanat".

Eine vorteilhafte Ausgestaltung der vorliegenden Erfindung ist auch ein Verfahren zur Herstellung einer Polyisocyanatzusammensetzung aus Toluylendiisocyanat, die eine nach DIN EN 1557:1997-03 bestimmte Farbzahl von < 100 Hazen, bevorzugt < 95 Hazen aufweist, umfassend die Zugabe mindestens eines gegenüber Isocyanatgruppen inerten Lösungsmittels zu mindestens einem Polyisocyanat aus Toluylendiisocyanat, dadurch gekennzeichnet, dass die Zugabe des Lösungsmittels in einer oder mehrerer Stufen erfolgt und mindestens eine dieser Stufen als kontinuierliche Verdünnung durchgeführt wird.

Vorliegend steht Toluylendiisocyanat als Oberbegriff für die Isomere 2,4-Toluylendiisocyanat, 2,6-Toluylendiisocyanat und beliebige Gemische aus 2,4- und 2,6-Toluylendiisocyanat.

Erfindungsgemäß bedeuten die Bezugnahmen auf "umfassend", "enthaltend" usw. bevorzugt "im Wesentlichen bestehend aus" und ganz besonders bevorzugt "bestehend aus".

Vorliegend ist unter "kontinuierlicher Verdünnung" zu verstehen, das mindestens zwei Volumenströme, bevorzugt genau zwei Volumenströme, derart miteinander vermischt werden, dass die Verdünnung im Wesentlichen ohne Konzentrationsverlauf erfolgt. Unter "im Wesentlichen ohne Konzentrationsverlauf" wird vorliegend verstanden, dass der Festkörpergehalt im abfließenden Produktstrom zwischen ≤ 10% über und ≤ 10% unter, bevorzugt zwischen ≤ 5% über und ≤ 5% unter und besonders bevorzugt zwischen ≤ 2% über und ≤ 2% unter dem zu erreichenden Festkörpergehalt des verdünnten Produktstroms variieren kann.

Geeignete Vorrichtungen zur kontinuierlichen Verdünnung sind beispielsweise T-Stücke, Zweileitungssysteme mit Statikmischer sowie Kessel bzw. Zwischenlösekessel. Als Kessel bzw. Zwischenlösekessel wird vorliegend ein Behälter verstanden, in den mindestens zwei Volumenströme kontinuierlich in ein gerührtes Volumen gegeben werden, aus dem der verdünnte Produktstrom entsprechend abfließt. Dieser Produktstrom stellt die erfindungsgemäße Polyisocyanat-zusammensetzung dar, wobei beim Vorliegen mehrerer Verdünnungsstufen der, nach der letzten Verdünnungsstufe erhaltene Produktstrom die erfindungsgemäße Polyisocyanatzusammensetzung darstellt.

Bevorzugt erfolgt die kontinuierliche Verdünnung dadurch, dass mindestens zwei Flüssigkeitsströme, besonders bevorzugt genau zwei Flüssigkeitsströme, kontinuierlich in ein gerührtes Volumen gegeben werden, aus dem der verdünnte Produktstrom bevorzugt kontinuierlich abfließt.

Ein solches gerührtes Volumen kann beispielsweise ein vorstehend genannter Kessel sein. In der Regel handelt es sich bei diesen Flüssigkeitsströmen zum einen um das zu lösende Polyisocyanat aus TDI (Polyisocyanatstrom) und zum anderen um das mindestens eine gegenüber Isocyanatgruppen inerte Lösungsmittel (Lösungsmittelstrom).

Im Gegensatz zur bekannten batch-Verdünnung können Nachteile bei der Durchmischung beim erfindungsgemäßen Verfahren weitestgehend vermieden werden, da stets direkt die gewünschte Konzentration vorliegt. Außerdem kann so die Verweilzeit so kurz wie möglich gehalten werden, was sich vorteilhaft auf die Stabilität der Produkte auswirkt.

Als Lösungsmittel können in der Polyurethanchemie gebräuchliche Verdünnungsmittel und Lösungsmittel wie beispielsweise Toluol, Xylol, Cyclohexan, Butylacetat, Ethylacetat, Ethylglykolacetat, Pentylacetat, Hexylacetat, Methoxypropylacetat, Tetrahydrofuran, Dioxan, Aceton, N-Methylpyrrolidon, Methylethylketon, Testbenzin, höher substituierte Aromaten, wie beispielsweise unter der Bezeichnung Solvent Naphtha^{®}, Solvesso^{®}, Shellsol^{®}, Isopar^{®}, Nappar^{®} und Diasol^{®} im Handel, Schwerbenzol, Tetralin, Dekalin und Alkane mit mehr als 6 Kohlenstoffatomen, übliche Weichmacher, wie Phthalate, Sulfonsäureester und Phosphorsäureester sowie Gemische derartiger Verdünnungs- und Lösungsmittel eingesetzt werden.

Ferner geeignet als Lösungsmittel sind auch Polyisocyanate auf Basis aliphatischer Diisocyanate wie sie z.B. in der DE-A 4 428 107 beschrieben sind. Hiermit sind verdünnte monomerenarme TDI-Trimerisate zugänglich, die keine oder weniger leicht verdampfbaren Lösungsmittel und Verdünnungsmittel enthalten.

In einer weiteren bevorzugten Ausführungsform des erfindungsgemäßen Verfahrens erfolgt die Zugabe des Lösungsmittels mindestens zweistufig, wobei die erste Stufe als kontinuierliche Verdünnung durchgeführt wird. Die mindestens erfolgende zweite Stufe kann kontinuierlich oder diskontinuierlich durchgeführt werden. Hieraus ergibt sich der Vorteil, dass die Trübungsstabilität der erfindungsgemäßen Polyisocyanatzusammensetzung noch weiter erhöht wird. Zusätzlich wird auch die Farbzahl weiter reduziert.

Es ist auch möglich, dass eine dritte, vierte, fünfte oder vielfache Stufe erfolgt, wobei hier zwischen erforderlichem verfahrenstechnischem Aufwand und der möglichem Zugewinn an weiterer Trübungsstabilität abgewogen werden muss. Hierbei hat sich herausgestellt, dass in den meisten Fällen eine zweistufige Zugabe die optimale Balance aus verfahrenstechnischem Aufwand und möglichem Zugewinn an weiterer Trübungsstabilität bringt.

Bei einer mehrstufigen bzw. mehrere Stufen umfassenden Zugabe mindestens eines gegenüber Isocyanatgruppen inerten Lösungsmittels werden in den sequenziell, in verschiedenen Vorrichtungen, wie beispielsweise Zwischenlösekesseln, ablaufenden Stufen verschiedene Verdünnungseinstellungen (Festkörpergehalte) erreicht. Diese Verdünnungseinstellungen werden im Folgenden auch als Festkörpergehalte bezeichnet. Vorliegend wird unter Festkörpergehalt der Gewichtsanteil des Polyisocyanats in der Polyisocyanatzusammensetzung verstanden.

In einer weiteren bevorzugten Ausführungsform des erfindungsgemäßen Verfahrens wird in der ersten Stufe ein Festkörpergehalt von ≥ 30 bis ≤ 90 Gew.-%, bevorzugt ≥ 50 bis ≤ 85 Gew.-%, besonders bevorzugt ≥ 55 bis ≤ 75 Gew.-% und ganz besonders bevorzugt ≥ 60 bis ≤ 70 Gew.-%, eingestellt.

Bei dieser mindestens zweistufig erfolgenden Zugabe ist es weiter bevorzugt, wenn in der zweiten Stufe ein Festkörpergehalt von ≥ 10 bis ≤ 80 Gew.-%, bevorzugt ≥ 15 bis ≤ 65 Gew.-%, besonders bevorzugt ≥ 20 bis ≤ 50 Gew.-% und ganz besonders bevorzugt ≥ 25 bis ≤ 35 Gew.-%, eingestellt wird, wobei der in der ersten Stufe eingestellte Festkörpergehalt in der zweiten Stufe zumindest um 15 Gew.-%, bevorzugt zumindest um 25 Gew.-% erniedrigt wird. Hieraus ergibt sich der Vorteil, dass sich über die mindestens zweistufige Zugabe Festkörpergehalte einstellen lassen, die für eine spätere Applikation der nach dem erfindungsgemäßen Verfahren erhältlichen Polyisocyanatzusammensetzung breitestmöglich geeignet sind und trotzdem trübungsstabil sind.

Erfindungsgemäß wird das Polyisocyanat aus Toluylendiisocyanat durch
(i) Umsetzung von Toluylendiisocyanat unter Bildung eines Polyisocyanates und
(ii) Entfernung des nicht umgesetzten Toluylendiisocyanates hergestellt.

Im Rahmen der vorliegenden Erfindung ist die "Entfernung des nicht umgesetzten Toluylendiisocyanates" als im Wesentlichen vollständig zu verstehen. Hierbei ist unter "im Wesentlichen vollständig" zu verstehen, dass sich Restgehalte an monomerem Toluylendiisocyanat von ≤ 0,5 Gew.-%, bevorzugt ≤ 0,3 Gew.-% und besonders bevorzugt ≤ 0,1 Gew.-%, bezogen auf das Gesamtgewicht des Polyisocyanats aus Toluylendiisocyanat ergeben.

Die Entfernung des nicht umgesetzten Toluylendiisocyanates in Schritt (ii) kann nach beliebigen Methoden erfolgen. Es ist jedoch bevorzugt, wenn die Entfernung des nicht umgesetzten Toluylendiisocyanates in Schritt (ii) durch mindestens ein thermisches Trennverfahren, welches eine oder mehrere Stufen aufweisen kann, bevorzugt durch mindestens ein zweistufiges thermisches Trennverfahren und besonders bevorzugt durch mindestens einen Fallrohrverdampfer und/oder mindestens einen Dünnschichtverdampfer erfolgt. Dies führt zu dem Vorteil, dass eine ausreichende Abtrennung des nicht umgesetzten Toluylendiisocyanates auch bei größeren Durchsätzen erreicht werden kann.

Geeignete thermische Trennverfahren sind beispielsweise Destillationen im Vakuum mittels Dünnschichtverdampfer und/oder Fallrohrverdampfer. Für die Entfernung von TDI sind im Allgemeinen Drücke im Bereich von 0,1-20 mbar und Temperaturen von 120-250 °C geeignet.

Bevorzugt wird das thermische Trennverfahren bei einer Heizmitteltemperatur von ≥ 140 bis ≤ 235 °C und bevorzugt von ≥ 160 bis ≤ 215 °C durchgeführt. Hieraus ergibt sich der Vorteil, dass die Entfernung des nicht umgesetzten Toluylendiisocyanats schonend und dennoch effizient erfolgt. Je nach verfahrenstechnischem Aufwand lassen sich Gehalte an monomerem Toluylendiisocyanat von ≤ 0,5 Gew.-%, bevorzugt ≤ 0,3 Gew.-% und besonders bevorzugt ≤ 0,1 Gew.-%, bezogen auf das Gesamtgewicht des Polyisocyanats aus Toluylendiisocyanat realisieren, je niedriger diese Gehalte sind, desto breiter ist die erfindungsgemäße Polyisocyanatzusammensetzung einsetzbar, da die Arbeitsplatzhygiene, insbesondere in handwerklichen Applikationen, noch weiter verbessert wird. Bestimmen lassen sich die Gehalte an nicht umgesetzten Toluylendiisocyanat gaschromatographisch nach DIN EN ISO 10283:2007-11 mit internem Standard.

Falls neben dem Toluylendiisocyanat noch weitere Diisocyanate aus der oben genannten Liste eingesetzt werden, so beziehen sich die vorstehend genannten Restgehalte und Abtrennmöglichkeiten auf die insgesamt vorhandenen Restgehalte an den gesamten monomeren Diisocyanaten, wobei der Fachmann beispielsweise hinsichtlich der Verfahrensparameter geringfügige Anpassungen vornehmen kann, um die Abtrennung auf das entsprechende zu entfernende monomere Diisocyanat abzustimmen.

In einer weiteren bevorzugten Ausführungsform wird in Schritt (i) des Verfahrens ein Gemisch aus 2,4- und 2,6-Toluylendiisocyanat eingesetzt, welches zu ≥ 50 bis ≤ 99 Gew.-%, bevorzugt zu ≥ 70 bis ≤ 95 Gew.-% und besonders bevorzugt zu ≥ 75 bis ≤ 90 Gew.-%, bezogen auf das Gesamtgewicht des eingesetzten Toluylendiisocyanates, aus 2,4-Toluylendiisocyanat besteht. Hieraus ergibt sich der weitere Vorteil, dass eine ausgewogene Balance zwischen Selektivität der unterschiedlich reaktiven Isocyanatgruppen im 2,4-TDI und Erhöhung der Kristallisationsstabilität durch zumindest einen kleinen Anteil an 2,6-TDI erreicht wird.

Sowohl 2,4- und 2,6-Toluylendiisocyanat als auch deren Gemische sind allgemein kommerziell verfügbar. Sie können nach bekannten Verfahren, wie beispielsweise durch Phosgenierung des entsprechenden Toluylendiamins (TDA) in der Flüssigphase oder der Gasphase hergestellt werden.

Besonders bevorzugt sind Toluylendiisocyanate, die durch Gasphasenphosgenierung von TDA hergestellt werden, da ein solches Verfahren besonders effizient ist.

In einer weiteren Ausführungsform ist das in Schritt (i) des Verfahrens gebildete Polyisocyanat ein Urethanguppen aufweisendes Polyisocyanat aus Toluylendiisocyanat. Dieses wird vorzugsweise so hergestellt, dass Polyhydroxyverbindungen mit der 5- bis 10-fachen molaren Menge TDI umgesetzt werden. Hierbei sind geeignete niedermolekulare Polyhydroxyverbindungen zwei- bis vierwertige Alkohole mit einem Molekulargewicht von 62 bis 146 und/oder aus ihnen durch Addition von Ethylen- und/oder Propylenoxid hergestellte Polyetherpolyole in reiner Form oder als beliebige Gemische.

Als zwei- bis vierwertige Alkohole kommen zum Beispiel in Betracht Ethylenglykol, 1,2-Propylenglykol, 1,3-Propylenglykol, 1,2-Butandiol, 1,3-Butandiol, 1,4-Butandiol, 1,5-Pentandiol, Neopentylglykol, 1,6-Hexandiol, 2-Ethylhexandiol, Glycerin, Trimethylolpropan und Pentaerythrit.

Geeignete Polyetherpolyole weisen ein aus Hydroxylgruppengehalt und Hydroxylfunktionalität berechenbares Molekulargewicht von 106 bis 600, vorzugsweise 106 bis 470 auf. Bevorzugt werden Polyetherdiole und Polyethertriole eingesetzt. Diese Polyetherpolyole können in an sich bekannter Weise durch Alkoxylierung geeigneter zwei- bis vierfunktioneller Startermoleküle oder geeigneter Gemische von Startermolekülen erhalten werden, wobei bei der Alkoxylierung insbesondere Propylenoxid und/oder Ethylenoxid, gegebenenfalls im Gemisch nacheinander in beliebiger Reihenfolge zum Einsatz gelangen. Bevorzugt werden als Startermoleküle die oben genannten zweibis vierwertigen Alkohole eingesetzt. Ganz besonders bevorzugt werden Gemische aus Trimethylolpropan und Diethylenglykol eingesetzt.

In einer weiteren bevorzugten Ausführungsform ist das Polyisocyanat des erfindungsgemäßen Verfahrens ein Isocyanatgruppen aufweisendes Polyisocyanurat aus Toluylendiisocyanat, wobei die Umsetzung von Toluylendiisocyanat in Schritt (i) unter Bildung von Isocyanuratgruppen in Anwesenheit mindestens eines Katalysators erfolgt und bei einem Gehalt an Isocyanatgruppen von ≥ 30 bis ≤ 46 Gew.-%, bevorzugt von ≥ 34 bis ≤ 44 Gew.-% und besonders bevorzugt von ≥ 38 bis ≤ 42 Gew.-% durch Zugabe mindestens eines Katalysatorgiftes abgestoppt wird.

Bei dieser Ausführungsform werden die Schritte (i) und (ii) vorzugsweise in Anwesenheit von ≥ 0 bis < 1 Gew.-% bei Destillationsbedingungen inerten, flüssigen, mindestens 50 °C über dem Toluylendiisocyanat siedenden Destillationshilfsmitteln und/oder ≥ 0 bis < 1 Gew.-%, bezogen auf das Gesamtgewicht der in Schritt (i) eingesetzten Verbindungen, an Verbindungen, die eine oder mehrere Hydroxylgruppen aufweisen, durchgeführt.

Besonders bevorzugt ist hierbei, dass solche Destillationshilfsmittel in Mengen von ≥ 0 bis ≤ 0,5 Gew.-%, bevorzugt ≥ 0 bis ≤ 0,25 Gew.-% und besonders bevorzugt ≥ 0 bis ≤ 0,1 Gew.-%, bezogen auf das Gesamtgewicht der in Schritt (i) eingesetzten Verbindungen, vorhanden sind und/oder, dass die Verbindungen, die eine oder mehrere Hydroxylgruppen aufweisen in Mengen von ≥ 0 bis ≤ 0,8 Gew.-%, bevorzugt ≥ 0 bis ≤ 0,5 Gew.-%, besonders bevorzugt ≥ 0 bis ≤ 0,1 Gew.-%, bezogen auf das Gesamtgewicht der in Schritt (i) eingesetzten Verbindungen, vorhanden sind. In diesen Mengen gegebenenfalls vorhandene Destillationshilfsmittel und/oder in diesen Mengen gegebenenfalls vorhandene Verbindungen, die eine oder mehrere Hydroxylgruppen aufweisen, haben keinen negativen Einfluss auf das erfindungsgemäße Verfahren. Jedoch ist es ganz besonders bevorzugt, wenn in den Schritten (i) und (ii) des erfindungsgemäßen Verfahrens keine Destillationshilfsmittel und/oder keine Verbindungen, die eine oder mehrere Hydroxylgruppen aufweisen, vorhanden sind, mit Ausnahme der gegebenenfalls vorhandenen, nachfolgend als Katalysatorbestandteil genannten aromatischen Hydroxylgruppen.

Für den Fall, dass gegenüber Isocyanatgruppen inerte Lösungsmittel bei dem erfindungsgemäßen Verfahren in den Schritten (i) bis (ii) zugegen sind, ist bevorzugt, dass solche Lösungsmittel in den Schritten (i) bis (ii) zu ≥ 0 bis ≤ 3 Gew.-%, bevorzugt ≥ 0 bis ≤ 1 Gew.-% und besonders bevorzugt ≥ 0 bis ≤ 0,05 Gew.-%, bezogen auf das Gesamtgewicht der in Schritt (i) eingesetzten Verbindungen, anwesend sein können.

Als Katalysatoren für die Bildung von Isocyanuratgruppen, folgend auch als Trimerisierungskatalysatoren bezeichnet, kommen grundsätzlich alle bekannten Katalysatoren des Standes der Technik wie beispielsweise Phosphine, Alkalisalze, Alkalialkoholate, tertiäre Amine, Fluoride, Hydrogendifluoride oder Hydrogenpolyfluoride in Betracht. Vorzugsweise werden Katalysatoren eingesetzt, die an Aromaten gebundene N,N-Dialkylaminomethylgruppen und phenolische OH-Gruppen aufweisen (Alkyl: unabhängig Alkylkette oder Alkylenkette mit bis 18 Kohlenstoffatomen, die gegebenenfalls durch Sauerstoff oder Schwefel getrennt sind). Diese Gruppen können auf mehrere Moleküle verteilt oder an einem oder mehreren benzolischen Aromaten positioniert sein. Besonders bevorzugt werden Katalysatoren eingesetzt, die sowohl Hydroxyl- als auch Dialkylaminomethylgruppen in einem Molekül enthalten. Ganz besonders bevorzugt werden Katalysatoren eingesetzt, deren Dialkylaminomethylgruppen (Alkyl = C₁ bis C₃-Kette) in ortho-Stellung zu aromatischen Hydroxylgruppen positioniert sind. Als Beispiele seien folgende Mannichbasen genannt, wie sie z.B. auf Basis von Phenol, p-Isononylphenol oder Bisphenol A zum Beispiel durch Umsetzung von 188 Gewichtsteilen Phenol mit 720 Teilen einer 25%igen wässrigen Dimethylamin-Lösung und 425 Gewichtsteilen 40%iger Formaldehyd-Lösung durch zweistündiges Erhitzen auf 80°C, Abtrennen der wässrigen Phasen und Destillation der organischen Phase bei 90°C/1.33 KPa(10 Torr) nach der DE-A 2 452 531 9 erhalten werden. Die Umsetzung in Schritt (i) erfolgt im Allgemeinen bei Temperaturen zwischen 20 und 120°C, bevorzugt zwischen 40 und 100°C und besonders bevorzugt zwischen 60 und 90°C.

Die Katalysatoren werden als Reinsubstanz oder gelöst gegebenenfalls in mehreren kleinen Portionen in Schritt (i) eingesetzt, wobei die Menge über einen breiten Bereich variiert werden kann. Vorzugsweise beträgt die Menge an insgesamt eingesetztem Katalysator ≥ 0,001 bis ≤ 2,0 Gew.-%, bevorzugt ≥ 0,003 bis ≤ 0,5 Gew.-% und besonders bevorzugt ≥ 0,005 bis ≤ 0,05 Gew.-%, bezogen auf das Gesamtgewicht der in Schritt (i) und (ii) eingesetzten Verbindungen.

Das Abstoppen der Umsetzung in Schritt (i) erfolgt durch Zugabe mindestens eines Katalysatorengifts, wobei als Katalysatorengifte beispielsweise Schwefel (im Falle der Verwendung von Phosphinen als Katalysatoren) oder Alkylierungsmittel wie beispielsweise Toluolsulfonsäuremethylester (bei der bevorzugten Verwendung von Mannich-Basen als Katalysatoren) oder auch Acylierungsmittel wie beispielsweise Benzoylchlorid Verwendung finden können.

Die Menge des einzusetzenden Katalysatorgiftes wird entsprechend der eingesetzten Katalysatormenge ausgewählt, so dass der Katalysator deaktiviert wird. Vorzugsweise wird eine insgesamt unteräquimolare Menge des Katalysatorgifts bezogen auf die Äquivalente an Lewis-Basen der Katalysatoren eingesetzt, können auch bereits > 20 bis < 100%, bezogen auf die eingesetzten Äquivalente an Lewis-Base des Katalysators, für eine vollständige Deaktivierung des Katalysators ausreichend sein.

Unabhängig davon welches Polyisocyanat aus TDI in dem erfindungsgemäßen Verfahren eingesetzt wird, erfolgt in einer weiteren bevorzugten Ausführungsform nach Zugabe des mindestens einen gegenüber Isocyanatgruppen inerten Lösungsmittels zu dem mindestens einem Polyisocyanat, bevorzugt aus Toluylendiisocyanat, in einem weiteren Schritt die Zugabe mindestens einer vom Polyisocyanat der vorstehenden Ausführungsformen verschiedenen Polyisocyanatzusammensetzung aus Toluylendiisocyanat, bevorzugt mindestens einer Polyisocyanuratzusammensetzung aus Toluylendiisocyanat und/oder mindestens einer Polyurethanzusammensetzung aus Toluylendiisocyanat, sowie optional die Zugabe eines oder mehrerer Hilfs- und Zusatzstoffe. Diese Polyisocyanuratzusammensetzung aus Toluylendiisocyanat und diese Polyurethanzusammensetzung aus Toluylendiisocyanat lassen sich nach bekannten Verfahren herstellen, aber auch nach den vorstehend beschriebenen Verfahren zur Herstellung des Isocyanatgruppen aufweisenden Polyisocyanurats aus Toluylendiisocyanat und des Urethanguppen aufweisendes Polyisocyanat aus Toluylendiisocyanat.

Hieraus ergibt sich der weitere Vorteil, dass die physikalischen und chemischen Eigenschaften von Gemischen, enthaltend mindestens ein erfindungsgemäßes Polyisocyanat, gezielt einstellen lassen.

Geeignete Hilfs- und Zusatzstoffe sind beispielsweise die üblichen Benetzungsmittel, Verlaufsmittel, Hautverhinderungsmittel, Antischaummittel, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden.

Eine Polyisocyanatzusammensetzung, hergestellt oder herstellbar nach dem erfindungsgemäßen Verfahren wird offenbart. Es hat sich überraschenderweise gezeigt, dass eine solche Verfahrensführung zu trübungsstabilen Zusammensetzungen führt, wohingegen eine Zugabe von Lösungsmittel, bei der nicht mindestens eine Stufe als kontinuierliche Verdünnung durchgeführt wird zu getrübten Zusammensetzungen führt.

Bei diesem Gegenstand der Offenbarung gilt dies bevorzugt für Polyisocyanatzusammensetzungen, bei denen das Polyisocyanat auf Basis von 1,5-Diisocyanatopentan (PDI), 2-Methyl-1,5-diisocyanatopentan, 1,5-Diisocyanato-2,2-dimethylpentan, 1,10-Diisocyanatodecan, 2,4- und 2,6-Diisocyanato-1-methylcyclohexan, 2,4'-Diisocyanatodicyclohexylmethan, 1-Isocyanato-1-methyl-4(3)isocyanato-methylcyclohexan, Bis-(isocyanatomethyl)-norbornan, 1,3- und 1,4-Bis(isocyanatomethyl)benzol (XDI), 1,3- und 1,4-Bis-(2-isocyanato-prop-2-yl)-benzol (TMXDI), Toluylendiisocyanat (TDI), 2,4'- und 4,4'-Diisocyanatodiphenylmethan (MDI), 1,5-Diisocyanatonaphthalin oder 1,3- und 1,4-Phenylendiisocyanat oder Mischungen der vorgenannten Diisocyanate ist. Besonders bevorzugt ist das Polyisocyanat auf Basis von 1,5-Diisocyanatopentan (PDI), 2-Methyl-1,5-diisocyanatopentan, 1,5-Diisocyanato-2,2-dimethylpentan, 1,10-Diisocyanatodecan, 2,4- und 2,6-Diisocyanato-1-methylcyclohexan, 2,4'-Diisocyanatodicyclohexylmethan, 1-Isocyanato-1-methyl-4(3)isocyanato-methylcyclohexan, Bis-(isocyanatomethyl)-norbornan, 1,3- und 1,4-Bis(isocyanatomethyl)benzol (XDI), 1,3- und 1,4-Bis-(2-isocyanato-prop-2-yl)-benzol (TMXDI), Toluylendiisocyanat (TDI), 2,4'- und 4,4'-Diisocyanatodiphenylmethan (MDI), 1,5-Diisocyanatonaphthalin oder 1,3- und 1,4-Phenylendiisocyanat ist und ganz besonders bevorzugt aus Toluylendiisocyanat (TDI).

Neben der vorteilhaften Trübungsstabilität weisen die offenbarungsgemäßen Polyisocyanatzusammensetzungen eine sehr geringe Farbzahl von < 100 Hazen, bevorzugt < 95 Hazen auf. Die Farbzahl in Hazen wird nach DIN EN 1557:1997-03 bestimmt.

Ein weiterer Gegenstand der Erfindung ist die Verwendung der kontinuierlichen Verdünnung bei der Lösung von Polyisocyanaten aus Toluylendiisocyanat, zur Verhinderung von Trübungen in der Polyisocyanatzusammensetzung.

Die erfindungsgemäße Polyisocyanatzusammensetzung ist sehr gut zur Verwendung als Vernetzungsmittel in einem Klebstoff oder in einem Beschichtungsmittel, bevorzugt in einem Klebstoff geeignet.

Die durch das erfindungsgemäße Verfahren herstellbaren Polyisocyanatzusammensetzungen werden vorzugsweise zur Herstellung von unter dem Einfluss von Luftfeuchtigkeit aushärtbaren Klebstoffen oder Beschichtungsmaterialien verwendet. Sie können ebenfalls zur Herstellung von Haftvermittlern, Druckfarben und Polyurethanformkörpern Verwendung finden. Besonders bevorzugt werden sie als Vernetzer in Zwei-Komponenten-Systemen mit an sich bekannten gegenüber Isocyanatgruppen reaktiven Verbindungen eingesetzt.

Ein weiterer Gegenstand der Offenbarung, aber nicht unter den Schutzumfang der Ansprüche fallend, ist daher ein Zwei-Komponenten-System, umfassend eine Isocyanatkomponente A), enthaltend mindestens eine offenbarungsgemäße Polyisocyanatzusammensetzung, und eine gegenüber Isocyanatgruppen reaktive Komponente B), enthaltend mindestens eine gegenüber Isocyanatgruppen reaktive Verbindung, bevorzugt mindestens einen Hydroxylgruppen aufweisenden Polyester.

Geeignete gegenüber Isocyanatgruppen reaktive Verbindungen sind beispielsweise hydroxyfunktionelle Polyether, -ester, -amide, -carbonate, -acrylate, -butadiene bzw. Mischtypen der genannten hydroxyfunktionellen Polymeren. Auch niedermolekulare Di- und Polyole, Di- und Trimerfettalkohole sowie aminofunktionelle Verbindungen können in dem erfindungsgemäßen Zwei-Komponenten-System Verwendung finden. Außerdem sind auch Cyclohexanon-Formaldehyd-Kondensate, beispielsweise in Ricinusöl, geeignet. Hydroxylgruppen aufweisende Polyester sind jedoch besonders bevorzugt. Zusätzlich können in den Beschichtungen oder Verklebungen auch andere Hilfs- und Zusatzstoffe wie beispielsweise die üblichen Benetzungsmittel, Verlaufsmittel, Hautverhinderungsmittel, Antischaummittel, Haftvermittler, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden. Die Beschichtungsmittel können in Form von Klarlacken als auch in Form pigmentierter Lacke verwendet werden.

Die erhaltenen Beschichtungsmaterialien oder Klebstoffe können zur Beschichtung oder Verklebung beliebiger Substrate wie beispielsweise natürliche oder künstliche Faserstoffe, bevorzugt Holz, Kunststoffe, Leder, Papier, Textilien, Glas, Keramik, Putz, Mauerwerk, Metalle oder Beton und besonders bevorzugt Papier oder Leder verwendet werden. Sie lassen sich mit üblichen Applikationsmethoden wie Spritzen, Streichen, Fluten, Gießen, Tauchen, Walzen aufbringen.

Daher ist ein Verbundsystem, hergestellt oder herstellbar durch Aushärten des auf mindestens ein Substrat aufgetragenen erfindungsgemäßen Zwei-Komponenten-Systems ein weiterer Gegenstand der Offenbarung, auch wenn es nicht unter den Schutzumfang der Ansprüche fällt.

Die Erfindung wird nachfolgend anhand von Beispielen und Vergleichsbeispielen näher erläutert.

### Beispiele

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909:2007-05.

Die Rest-Monomeren Gehalte wurden nach DIN EN ISO 10283:2007-11 gaschromatographisch mit internem Standard gemessen.

Die Trübung wurde nach DIN EN ISO 7027-1:2016 gemessen.

### Beispiel 1 (nicht erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das erhaltene heiße Harz (370 g) wird mittels einer Pumpe direkt in einen gerührten Kolben, ausgestattet mit einem Rückflusskühler, zu 863 g siedendem Ethylacetat gefördert, was einer einstufigen, diskontinuierlichen Verdünnung von 100% Festkörpergehalt auf 30% Festkörpergehalt entspricht. Man erhält eine Lösung mit folgenden Eigenschaften:
NCO: 7,1%
Monomerengehalt: 0,08%
Trübung: 21 NTU

### Beispiel 2 (erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das aus der Destillation ablaufende heisse Harz wird mit 105 g/h mittels einer Pumpe direkt in einen gerührten 500ml-4-Hals-Kolben, ausgestattet mit einem Rückflusskühler, gefördert. Zeitgleich läßt man aus einem Tropftrichter 245 g/h Ethylacetat zulaufen und überführt das kontinuierlich verdünnte Produkt über ein getauchtes Steigrohr mittels einer Pumpe mit 350 g/h kontinuierlich in einen gekühlten Vorlagekolben. Der Inhalt des 4-Hals-Kolbens bleibt bei ca. 400 g konstant hat eine Temperatur von ca. 80°C. Man erhält eine Lösung mit einem Festkörpergehalt von 30% und folgenden Eigenschaften:
NCO: 7,0%
Monomerengehalt: 0,03%
Trübung: 12 NTU

### Beispiel 3 (erfindungsgemäß):

1500 g einer Toluylendiisocyanat-Isomerenmischung aus ca. 80% 2,4-Toluylendiisocyanat und 20% 2,6- Toluylendiisocyanat werden bei 80°C in einem 2L-Kolben vorgelegt. Dann werden 0,52 g einer Mannichbase (Bisphenol/Formaldehyd/Dimethylamin 25%ig in Butylacetat / Xylol 19:56) innerhalb von 2 Stunden unter Rühren zudosiert, wobei die Temperatur bei 78-82°C gehalten wird. Wenn ein NCO-Gehalt 40,4% erreicht ist, wird 1 g Dibutylphosphat zum Abstoppen der Reaktion zugegeben. Aus der so erhaltenen Rohware wird das überschüssige Diisocyanat anschließend bei Temperaturen von 180°C und einem Druck von 0,05 mbar kontinuierlich destillativ entfernt. Das aus der Destillation ablaufende heisse Harz wird mit 105 g/h mittels einer Pumpe direkt in einen gerührten 500ml-4-Hals-Kolben, ausgestattet mit einem Rückflusskühler, gefördert. Zeitgleich läßt man aus einem Tropftrichter 57 g/h Ethylacetat zulaufen und überführt das kontinuierlich verdünnte Produkt über ein getauchtes Steigrohr mittels einer Pumpe mit 162 g/h kontinuierlich in einen gekühlten Vorlagekolben. Der Inhalt des 4-Hals-Kolbens bleibt bei ca. 400 g konstant hat eine Temperatur von ca. 80°C.

Anschließend wird der Inhalt des Vorlagekolbens in einem diskontinuierlichen Schritt mit Ethylacetat auf 30% Festkörper verdünnt. Man erhält eine Lösung mit folgenden Eigenschaften:
NCO: 7,0%
Monomerengehalt: 0,05%
Trübung: 2 NTU

## Patentansprüche

1. Verfahren zur Herstellung einer Polyisocyanatzusammensetzung, umfassend die Zugabe mindestens eines gegenüber Isocyanatgruppen inerten Lösungsmittels zu mindestens einem Polyisocyanat, **dadurch gekennzeichnet, dass** die Zugabe des Lösungsmittels in einer oder mehrerer Stufen erfolgt und mindestens eine dieser Stufen als kontinuierliche Verdünnung durchgeführt wird und wobei das Polyisocyanat auf Basis von Toluylendiisocyanat ist und durch
(i) Umsetzung von Toluylendiisocyanat unter Bildung eines Polyisocyanates und
(ii) Entfernung des nicht umgesetzten Toluylendiisocyanates bis zu einem Restgehalt an monomerem Toluylendiisocyanat von ≤ 0,5 Gew.-%, bezogen auf das Gesamtgewicht des Polyisocyanats aus Toluylendiisocyanat, hergestellt wird.

2. Verfahren gemäß Anspruch 1, **dadurch gekennzeichnet, dass** die Zugabe des Lösungsmittels mindestens zweistufig erfolgt, wobei die erste Stufe als kontinuierliche Verdünnung durchgeführt wird.

3. Verfahren gemäß Anspruch 1 oder 2, **dadurch gekennzeichnet, dass** in der ersten Stufe ein Festkörpergehalt von ≥ 30 bis ≤ 90 Gew.-%, bevorzugt ≥ 50 bis ≤ 85 Gew.-%, besonders bevorzugt ≥ 55 bis ≤ 75 Gew.-% und ganz besonders bevorzugt ≥ 60 bis ≤ 70 Gew.-%, eingestellt wird.

4. Verfahren gemäß Anspruch 2 oder 3, **dadurch gekennzeichnet, dass** in der zweiten Stufe ein Festkörpergehalt von ≥ 10 bis ≤ 80 Gew.-%, bevorzugt ≥ 15 bis ≤ 65 Gew.-%, besonders bevorzugt ≥ 20 bis ≤ 50 Gew.-% und ganz besonders bevorzugt ≥ 25 bis ≤ 35 Gew.-%, eingestellt wird, wobei der in der ersten Stufe eingestellte Festkörpergehalt in der zweiten Stufe zumindest um 15 Gew.-%, bevorzugt zumindest um 25 Gew.-% erniedrigt wird.

5. Verfahren gemäß einem der vorstehenden Ansprüche, **dadurch gekennzeichnet, dass** die Entfernung des nicht umgesetzten Toluylendiisocyanates in Schritt (ii) bis zu einem Restgehalt an monomerem Toluylendiisocyanat von ≤ 0,3 Gew.-% und besonders bevorzugt ≤ 0,1 Gew.-%, bezogen auf das Gesamtgewicht des Polyisocyanats aus Toluylendiisocyanat, erfolgt.

6. Verfahren gemäß einem der vorstehenden Ansprüche, **dadurch gekennzeichnet, dass** die Entfernung des nicht umgesetzten Toluylendiisocyanates in Schritt (ii) durch mindestens ein thermisches Trennverfahren, bevorzugt durch mindestens ein zweistufiges thermisches Trennverfahren und besonders bevorzugt durch mindestens einen Fallrohrverdampfer und/oder mindestens einen Dünnschichtverdampfer erfolgt.

7. Verfahren gemäß Anspruch 6, **dadurch gekennzeichnet, dass** das mindestens eine thermische Trennverfahren bei einer Heizmitteltemperatur von ≥ 140 bis ≤ 235 °C und bevorzugt von ≥ 160 bis ≤ 215 °C durchgeführt wird.

8. Verfahren gemäß einem der vorstehenden Ansprüche, **dadurch gekennzeichnet, dass** das Polyisocyanat aus Toluylendiisocyanat ein Isocyanatgruppen aufweisendes Polyisocyanurat aus Toluylendiisocyanat ist, wobei die Umsetzung von Toluylendiisocyanat in Schritt (i) unter Bildung von Isocyanuratgruppen in Anwesenheit mindestens eines Katalysators erfolgt und bei einem Gehalt an Isocyanatgruppen von ≥ 30 bis ≤ 46 Gew.-%, bevorzugt von ≥ 34 bis ≤ 44 Gew.-% und besonders bevorzugt von ≥ 38 bis ≤ 42 Gew.-% durch Zugabe mindestens eines Katalysatorgiftes abgestoppt wird.

9. Verfahren gemäß einem der vorstehenden Ansprüche, **dadurch gekennzeichnet, dass** die Schritte (i) und (ii) in Anwesenheit von ≥ 0 bis < 1 Gew.-% bei Destillationsbedingungen inerten, flüssigen, mindestens 50 °C über Toluylendiisocyanat siedenden Destillationshilfsmitteln und/oder ≥ 0 bis < 1 Gew.-%, bezogen auf das Gesamtgewicht der in Schritt (i) und (ii) eingesetzten Verbindungen, an Verbindungen, die eine oder mehrere Hydroxylgruppen aufweisen, durchgeführt werden.

10. Verfahren gemäß einem der vorstehenden Ansprüche, **dadurch gekennzeichnet, dass** nach Zugabe des mindestens einen gegenüber Isocyanatgruppen inerten Lösungsmittels zu dem mindestens einen Polyisocyanat in einem weiteren Schritt die Zugabe mindestens einer vom Polyisocyanat der vorstehenden Ansprüche verschiedenen Polyisocyanatzusammensetzung aus Toluylendiisocyanat, bevorzugt mindestens einer Polyisocyanuratzusammensetzung aus Toluylendiisocyanat und/oder mindestens einer Polyurethanzusammensetzung aus Toluylendiisocyanat, sowie optionale Zugabe eines oder mehrerer Hilfs- und Zusatzstoffe erfolgt.

11. Verwendung der kontinuierlichen Verdünnung bei der Lösung von Polyisocyanaten aus Toluylendiisocyanat zur Verhinderung von Trübungen in der Polyisocyanatzusammensetzung.

## Claims

1. Process for producing a polyisocyanate composition comprising addition of at least one isocyanate-inert solvent to at least one polyisocyanate, **characterized in that** the addition of the solvent is carried out in one or more stages and at least one of these stages is performed as a continuous dilution and wherein the polyisocyanate is based on tolylene diisocyanate and is produced by
(i) reaction of tolylene diisocyanate to form a polyisocyanate and
(ii) removal of the unconverted tolylene diisocyanate down to a residual content of monomeric tolylene diisocyanate of ≤ 0.5% by weight based on the total weight of the polyisocyanate composed of tolylene diisocyanate.

2. Process according to Claim 1, **characterized in that** the addition of the solvent is carried out in at least two stages, wherein the first stage is performed as a continuous dilution.

3. Process according to Claim 1 or 2, **characterized in that** a solids content of ≥ 30% to ≤ 90% by weight, preferably ≥ 50% to ≤ 85% by weight, particularly preferably ≥ 55% to ≤ 75% by weight and very particularly preferably ≥ 60% to ≤ 70% by weight is established in the first stage.

4. Process according to Claim 2 or 3, **characterized in that** a solids content of ≥ 10% to ≤ 80% by weight, preferably ≥ 15% to ≤ 65% by weight, particularly preferably ≥ 20% to ≤ 50% by weight and very particularly preferably ≥ 25% to ≤ 35% by weight is established in the second stage, wherein the solids content established in the first stage is reduced by at least 15% by weight, preferably by at least 25% by weight, in the second stage.

5. Process according to any of the preceding claims, **characterized in that** the removal of the unconverted tolylene diisocyanate in step (ii) is carried out down to a residual content of monomeric tolylene diisocyanate of ≤ 0.3% by weight and particularly preferably ≤ 0.1% by weight based on the total weight of the polyisocyanate composed of tolylene diisocyanate.

6. Process according to any of the preceding claims, **characterized in that** the removal of the unconverted tolylene diisocyanate in step (ii) is carried out by means of at least one thermal separation process, preferably by means of at least one two-stage thermal separation process and particularly preferably by means of at least one falling film evaporator and/or at least one thin film evaporator.

7. Process according to Claim 6, **characterized in that** the at least one thermal separation process is performed at a heating medium temperature of ≥ 140°C to ≤ 235°C and preferably of ≥ 160°C to ≤ 215°C.

8. Process according to any of the preceding claims, **characterized in that** the polyisocyanate composed of tolylene diisocyanate is an isocyanate-containing polyisocyanurate composed of tolylene diisocyanate, wherein the reaction of tolylene diisocyanate in step (i) to form isocyanurate groups is carried out in the presence of at least one catalyst and is terminated at a content of isocyanate groups of ≥ 30% to ≤ 46% by weight, preferably of ≥ 34% to ≤ 44% by weight and particularly preferably of ≥ 38% to ≤ 42% by weight by addition of at least one catalyst poison.

9. Process according to any of the preceding claims, **characterized in that** the steps (i) and (ii) are performed in the presence of ≥ 0% to < 1% by weight of distillation aids that are inert under distillation conditions, liquid and have a boiling point at least 50°C higher than that of tolylene diisocyanate and/or ≥ 0% to < 1% by weight based on the total weight of the compounds employed in step (i) and (ii) of compounds comprising one or more hydroxyl groups.

10. Process according to any of the preceding claims, **characterized in that** addition of the at least one isocyanate-inert solvent to the at least one polyisocyanate is followed in a further step by addition of at least one polyisocyanate composition composed of tolylene diisocyanate which is distinct from the polyisocyanate of the preceding claims, preferably at least one polyisocyanurate composition composed of tolylene diisocyanate and/or at least one polyurethane composition composed of tolylene diisocyanate, and optionally by addition of one or more assistant and additive substances.

11. Use of continuous dilution in the dissolution of polyisocyanates composed of tolylene diisocyanate for preventing cloudiness in the polyisocyanate composition.

## Revendications

1. Procédé de préparation d'une composition de polyisocyanate, comprenant l'ajout d'au moins un solvant inerte vis-à-vis des groupes isocyanate à au moins un polyisocyanate, **caractérisé en ce que** l'ajout du solvant est effectué en une ou plusieurs étapes et au moins l'une de ces étapes est réalisée comme une dilution continue et le polyisocyanate étant à base de diisocyanate de toluylène et étant préparé par
(i) transformation de diisocyanate de toluylène avec formation d'un polyisocyanate et
(ii) élimination du diisocyanate de toluylène non transformé jusqu'à une teneur résiduelle en diisocyanate de toluylène monomère ≤ 0,5% en poids, par rapport au poids total du polyisocyanate basé sur le diisocyanate de toluylène.

2. Procédé selon la revendication 1, **caractérisé en ce que** l'ajout du solvant est effectué au moins en deux étapes, la première étape étant réalisée comme une dilution continue.

3. Procédé selon la revendication 1 ou 2, **caractérisé en ce qu'**on règle, dans la première étape, une teneur en solides ≥ 30 à ≤ 90% en poids, de préférence ≥ 50 à ≤ 85% en poids, de manière particulièrement préférée ≥ 55 à ≤ 75% en poids et de manière tout particulièrement préférée ≥ 60 à ≤ 70% en poids.

4. Procédé selon la revendication 2 ou 3, **caractérisé en ce qu'**on règle, dans la deuxième étape, une teneur en solides ≥ 10 à ≤ 80% en poids, de préférence ≥ 15 à ≤ 65% en poids, de manière particulièrement préférée ≥ 20 à ≤ 50% en poids et de manière tout particulièrement préférée ≥ 25 à ≤ 35% en poids, la teneur en solides réglée dans la première étape étant abaissée d'au moins 15% en poids, de préférence d'au moins 25% en poids, dans la deuxième étape.

5. Procédé selon l'une des revendications précédentes, **caractérisé en ce que** l'élimination du diisocyanate de toluylène non transformé dans l'étape (ii) est effectuée jusqu'à une teneur résiduelle en diisocyanate de toluylène monomère ≤ 0,3% en poids et de manière particulièrement préférée ≤ 0,1% en poids, par rapport au poids total du polyisocyanate basé sur le diisocyanate de toluylène.

6. Procédé selon l'une des revendications précédentes, **caractérisé en ce que** l'élimination du diisocyanate de toluylène non transformé dans l'étape (ii) est effectuée par au moins un procédé de séparation thermique, de préférence par au moins un procédé de séparation thermique en deux étapes et de manière particulièrement préférée par au moins un évaporateur à tuyau descendant et/ou au moins un évaporateur à couche mince.

7. Procédé selon la revendication 6, **caractérisé en ce que** ledit au moins un procédé de séparation est réalisé à une température d'agent caloporteur ≥ 140 à ≤ 235°C et de préférence ≥ 160 à ≤ 215°C.

8. Procédé selon l'une des revendications précédentes, **caractérisé en ce que** le polyisocyanate basé sur le diisocyanate de toluylène est un polyisocyanurate, présentant des groupes isocyanate, basé sur le diisocyanate de toluylène, la transformation du diisocyanate de toluylène dans l'étape (i) ayant lieu avec formation de groupes isocyanurate en présence d'au moins un catalyseur et étant arrêtée à une teneur en groupes isocyanate ≥ 30 à ≤ 46% en poids, de préférence ≥ 34 à ≤ 44% en poids et de manière particulièrement préférée ≥ 38 à ≤ 42% en poids par ajout d'au moins un poison de catalyseur.

9. Procédé selon l'une des revendications précédentes, **caractérisé en ce que** les étapes (i) et (ii) sont réalisées en présence de ≥ 0 à < 1% en poids d'adjuvants de distillation inertes et liquides aux conditions de distillation, présentant un point d'ébullition supérieur d'au moins 50°C à celui du diisocyanate de toluylène et/ou de ≥ 0 à < 1% en poids, par rapport au poids total des composés utilisés dans l'étape (i) et (ii), de composés qui présentent un ou plusieurs groupes hydroxyle.

10. Procédé selon l'une des revendications précédentes, **caractérisé en ce qu'**après l'ajout dudit au moins un solvant inerte vis-à-vis des groupes isocyanates audit au moins un polyisocyanate, dans une autre étape, on effectue l'ajout d'au moins une composition de polyisocyanate, différente du polyisocyanate des revendications précédentes, basée sur le diisocyanate de toluylène, de préférence l'ajout d'au moins une composition de polyisocyanurate basée sur le diisocyanate de toluylène et/ou l'ajout d'au moins une composition de polyuréthane basée sur le diisocyanate de toluylène, ainsi que l'ajout éventuel d'un ou de plusieurs adjuvants et additifs.

11. Utilisation de la dilution continue lors de la dissolution de polyisocyanates basés sur le diisocyanate de toluylène afin d'empêcher des troubles dans la composition de polyisocyanate.
